(19) Europäisches Patentamt
European Patent Office
Office européen des brevets

(11) **EP 2 101 648 B1**

(12) **EUROPÄISCHE PATENTSCHRIFT**

(45) Veröffentlichungstag und Bekanntmachung des Hinweises auf die Patenterteilung:
**25.05.2011 Patentblatt 2011/21**

(51) Int Cl.:
*A61B 6/02* (2006.01)     *A61B 6/00* (2006.01)
*G06T 5/40* (2006.01)

(21) Anmeldenummer: 07857317.7

(22) Anmeldetag: **10.12.2007**

(86) Internationale Anmeldenummer:
**PCT/EP2007/063592**

(87) Internationale Veröffentlichungsnummer:
**WO 2008/074681 (26.06.2008 Gazette 2008/26)**

(54) **VERFAHREN UND EINRICHTUNG ZUM ERZEUGEN EINES TOMOSYNTHETISCHEN 3D-RÖNTGENBILDES**

METHOD AND DEVICE FOR GENERATING A TOMOSYNTHETIC 3D X-RAY IMAGE

PROCÉDÉ ET DISPOSITIF DE FORMATION D'UNE IMAGE RADIOTOMOGRAPHIQUE DE SYNTHÈSE EN 3D

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **19.12.2006 DE 102006060039**

(43) Veröffentlichungstag der Anmeldung:
**23.09.2009 Patentblatt 2009/39**

(73) Patentinhaber: **Siemens Aktiengesellschaft**
**80333 München (DE)**

(72) Erfinder: **LUDWIG, Jasmina**
**81375 München (DE)**

(56) Entgegenhaltungen:
EP-A- 0 366 387          WO-A-96/21198
US-A- 5 165 100          US-A1- 2003 194 049
US-A1- 2005 135 559

• **FANG-FANG YIN ET AL: "COMPUTERIZED DETECTION OF MASSES IN DIGITAL MAMMOGRAMS: AUTOMATED ALIGNMENT OF BREAST IMAGES AND ITS EFFECT ON BILATERAL-SUBTRACTION TECHNIQUE" MEDICAL PHYSICS, AIP, MELVILLE, NY, US, Bd. 21, Nr. 3, 1. März 1994 (1994-03-01), Seiten 445-452, XP000435153 ISSN: 0094-2405**
• **DOBBINS J T ET AL: "Digital x-ray tomosynthesis: current state of the art and clinical potential" PHYSICS IN MEDICINE AND BIOLOGY, TAYLOR AND FRANCIS LTD. LONDON, GB, Bd. 48, Nr. 19, 7. Oktober 2003 (2003-10-07), Seiten R65-R106, XP002277335 ISSN: 0031-9155 in der Anmeldung erwähnt**

EP 2 101 648 B1

## Beschreibung

Die Erfindung bezieht sich auf ein insbesondere für die Mammographie geeignetes Verfahren zum Erzeugen eines tomosynthetischen Röntgenbildes von einem Untersuchungsobjekt, bei dem aus einer Mehrzahl von mit verschiedenen Projektionswinkeln aufgenommenen digitalen Einzelbildern ein tomosynthetisches 3D-Röntgenbild zusammengesetzt wird. Außerdem bezieht sich die Erfindung auf eine mit diesem Verfahren arbeitende Einrichtung.

Bei der Mammographie handelt es sich um eine Röntgenuntersuchung der weiblichen Brust mit dem Ziel, Tumore in einem möglichst frühen Stadium zu erkennen. Durch stetige Verbesserung der Mammographieverfahren wird angestrebt, Röntgenbilder mit hoher Aussagekraft zu erzeugen, um gutartige von bösartigen Veränderungen zu unterscheiden und die Zahl der fehlerhaften Befunde, d. h. die Zahl der verdächtigen Befunde, die von nicht bösartigen Veränderungen hervorgerufen sind, und die Zahl der nicht entdeckten bösartigen Tumore, zu reduzieren. Bei der herkömmlichen Röntgenmammographie wird dabei in einer einzigen Projektionsrichtung ein zweidimensionales Einzelbild der auf einer Lagerplatte gelagerten und mit einer Kompressionsplatte komprimierten Brust erzeugt. Da bei einer solchen Projektion die in Richtung des Röntgenstrahls hintereinander liegenden Gewebeschichten überlagert sind, können stark absorbierende gutartige Strukturen einen bösartigen Tumor überlagern und dessen Erkennbarkeit erschweren, oder es kann durch Überlagerung von Gewebestrukturen im ungünstigen Fall ein bösartiger Tumor vorgetäuscht werden.

Um dies zu vermeiden sind beispielsweise aus Dobbins JT III, Godfrey DJ, "Digital x-ray tomosynthesis: current state of the art and clinical potential", Physics in Medicine and Biology 48, R65-R106, 2003, als Tomosynthese bezeichnete Mammo-Mammographieverfahren bekannt, bei denen mit einem digitalen Röntgendetektor von der weiblichen Brust Einzelbilder oder Projektionsdaten in einer Mehrzahl von verschiedenen Projektionsrichtungen aufgenommen werden. Aus diesen unter verschiedenen Projektionswinkeln aufgenommenen digitalen Einzelbildern, d.h. aus den zu diesen Einzelbildern gehörenden Bilddaten, kann dann durch Bildrekonstruktionsverfahren ein dreidimensionaler Bilddatensatz erzeugt werden, der beispielsweise aus einer Mehrzahl von Schichtbildern besteht, die jeweils eine parallel zur Empfangsfläche des Röntgendetektors orientierte Schicht der Brust wiedergeben. Ein solcher durch Rekonstruktion gewonnener Bilddatensatz wird im Folgenden als tomosynthetisches 3D-Röntgenbild bezeichnet. Durch die Erzeugung eines solchen tomosynthetischen 3D-Röntgenbildes können in Ausbreitungsrichtung des Röntgenstrahls gesehen tiefer liegende Gewebsstrukturen besser erkannt werden.

Bei einem solchen bekannten tomosynthesefähigen Mammographiesystem wird bei ruhendem Röntgendetektor die Röntgenquelle in einem begrenzten Winkelbereich geschwenkt. Aufgrund dieses begrenzten Winkelbereiches ist eine exakte Rekonstruktion eines Volumenbildes nicht möglich, so dass die Volumenbilder Bildartefakte aufweisen können, die deren diagnostische Verwertbarkeit erschweren. Zur Verbesserung des durch Bildrekonstruktionsverfahren gewonnenen tomosynthetischen 3D-Röntgenbildes ist es beispielsweise aus der US 2005/0135559 A1 bekannt, die Einzel- oder Projektionsbilder einer Vorverarbeitung zu unterziehen, um auf diese Weise Streifenartefakte zu unterdrücken. Hierzu werden für die Projektionsbilder jeweils zweidimensionele Masken erzeugt, bei der jedem Pixel ein Gewichtsfaktor zugeordnet ist.

In den mit einem solchen Mammographiesystem erzeugten tomosynthetischen Schichtbildern werden jedoch neben solchen Streifenartefakten am Objektrand - die "Skinline" oder Brustgrenze - weitere, sogenannte Fächer-Artefakte beobachtet.

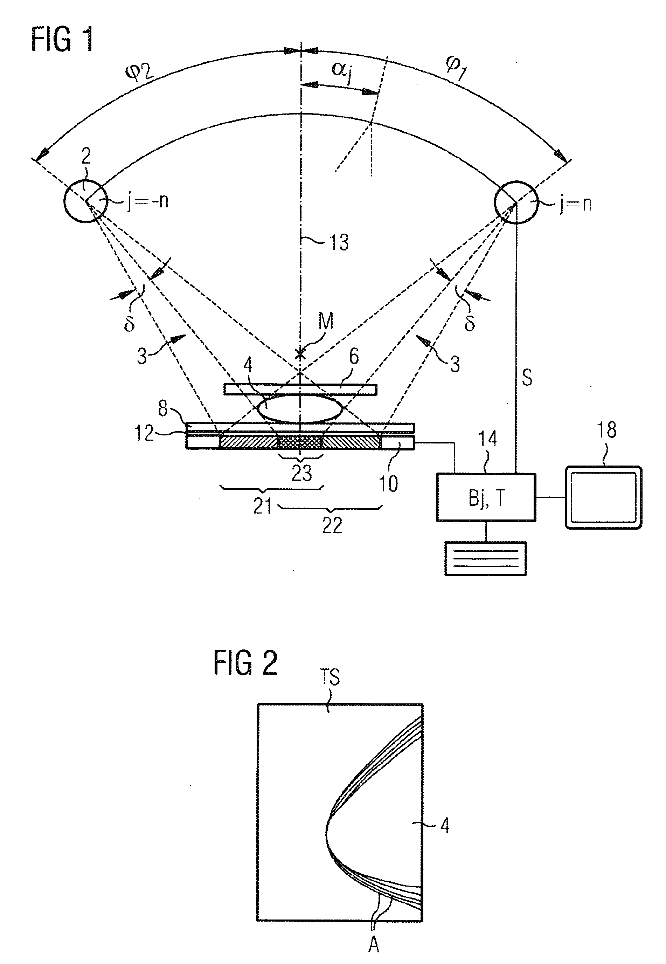

Diese Fächer-Artefakte treten vor allem in den Schichtbildern auf, die äußere Schichten des Untersuchungsobjektes, d.h. Schichten wiedergeben, die sich nahe am Detektor bzw. nahe an der Kompressionsplatte befinden. Ein solches von Fächer-Artefakten überlagertes tomosynthetisches Schichtbild TS ist in Fig. 2 dargestellt. In dieser Fig. ist zu erkennen, dass der Randbereich der im tomosynthetischen Schichtbild TS wiedergegebenen Schicht des Untersuchungsobjektes 4 von einer Vielzahl von feinen Linien A und dazwischen liegenden grauen Zonen umgeben ist, die eine Festlegung der Grenze des Untersuchungsobjektes 4 erschweren. Die Anzahl der zwischen diesen Linien A gebildeten Fächer entspricht dabei der Anzahl der Projektionen, die zur Rekonstruktion des tomosynthetischen 3D-Röntgenbildes herangezogen werden.

Diese Fächer-Artefakte lassen sich in den fertigen tomosynthetischen Schichtbildern, d. h. im fertigen tomosynthetischen 3D-Röntgenbild mit herkömmlichen Methoden der digitalen Bildverarbeitung allein auf der Grundlage der Grauwertinformation nicht oder nur mit sehr unbefriedigendem Ergebnis eliminieren. In der Praxis werden deshalb diese durch die Fächer-Artefakte gebildeten Bildbereiche manuell aus den Schichtbildern entfernt.

Der Erfindung liegt nun die Aufgabe zugrunde, ein Verfahren zum Erzeugen eines tomosynthetischen 3D-Röntgenbildes anzugeben, mit dem das Auftreten von Fächer-Artefakten automatisch unterdrückt wird. Außerdem liegt der Erfindung die Aufgabe zugrunde, eine nach diesem Verfahren arbeitende Einrichtung anzugeben.

Hinsichtlich des Verfahrens wird die genannte Aufgabe gelöst mit einem Verfahren mit den Merkmalen des Patentanspruches 1. Bei diesem Verfahren zum Erzeugen eines tomosynthetischen 3D-Röntgenbildes wird von einem Untersuchungsobjekt mit verschiedenen Projektionswinkeln in einem begrenzten Winkelbereich mit einer Röntgenquelle und einem digitalen Röntgendetektor eine Mehrzahl von digitalen 2D-Röntgenbildern aufgenommen, aus denen das tomosynthetische 3D-Röntgenbild durch eine Rückprojektion ermittelt wird, wobei zusätzlich folgenden Verfahrensschritte durchgeführt werden:

a) Aus den Bilddaten eines mit einem ersten Projektionswinkel aufgenommenen ersten 2D-Röntgenbildes und den Bilddaten eines mit einem zweiten Projektionswinkel aufgenommenen zweiten 2D-Röntgenbildes wird durch Rückprojektion jedem Voxel ein erster bzw. zweiter Voxelwert zugeordnet,

b) aus den ersten und zweiten Voxelwerten wird ein 3D-Datensatz erzeugt, bei dem jedem Voxel ein dritter Voxelwert zugeordnet ist, der dadurch festgelegt ist, dass jedem Voxel als dritter Voxelwert der erste Voxelwert zugeordnet wird, wenn dieser größer als der zweite Voxelwert ist, und jedem Voxel als dritter Voxelwert der zweite Voxelwert zugeordnet wird, wenn dieser größer als der erste Voxelwert ist,

c) aus der Häufigkeitsverteilung (dem Histogramm) der dritten Voxelwerte wird ein Schwellwert ermittelt, mit dem eine erste binäre 3D-Maske für das tomosynthetische 3D-Röntgenbild gebildet wird, die für alle Voxel den Wert 0 erhält, deren dritter Voxelwert den Schwellwert überschreitet,

d) das tomosynthetische 3D-Röntgenbild wird unter Zuhilfenahme der ersten binären 3D-Maske oder einer aus dieser durch Methoden der Bildverarbeitung abgeleiteten, gefilterten oder geglätteten zweiten binären 3D-Maske erzeugt.

Durch diese Maßnahmen ist es möglich, ein tomosynthetisches 3D-Röntgenbild oder gleichbedeutend damit eine Mehrzahl von 2D-Schichtbildern zu erzeugen, die weitgehend frei von Fächer-Artefakten sind, da durch die mit dem erfindungsgemäßen Verfahren erzeugte erste oder zweite binäre 3D-Maske außerhalb des Untersuchungsobjektes liegende Detektorbereiche automatisch ausgeblendet werden können.

Durch eine solche automatische Segmentierung des Objektbereiches und des objektfreien Detektorbereiches ist es außerdem möglich, die objektfreien Detektorbereiche, d.h. die Direktstrahlungsbereiche bei der Wiedergabe der Schichtbilder auf einem Monitor mit einem einheitlichen Grauwert - beispielsweise Schwarz - wiederzugeben. Dadurch wird für den Betrachter die Erkennbarkeit diagnostisch relevanter feiner Strukturen im Objektbereich erleichtert.

Darüber hinaus ist es durch eine solche Segmentierung, d.h. ein Ausblenden des objektfreien Detektorbereiches möglich, den Objektbereich einer separaten Bildbearbeitung zu unterziehen, die durch das Fehlen des objektfreien Detektorbereiches zu qualitativ besseren Ergebnissen führen kann.

Wenn für die Ermittlung des tomosynthetischen 3D-Röntgenbildes anstelle der ersten binären 3D-Maske eine durch Methoden der Bildverarbeitung berechnete zweite binäre 3D-Maske verwendet wird, kann die Bildqualität zusätzlich verbessert werden. Hierzu stehen gängige Methoden der Bildverarbeitung zur Verfügung, beispielsweise Erosion oder Dilatation sowie Kantenfilter, um fehlerhafte Bereiche der ersten 3D-Maske zu eliminieren und den Maskenrand zu glätten.

Mit Hilfe einer solcher binären 3D-Maske ist es möglich, ein gemäß dem Stand der Technik rekonstruiertes und somit artefaktbehaftetes tomosynthetisches 3D-Roh-Röntgenbild nachträglich zu bearbeiten und auf diese Weise von den Fächer-Artefakten zu befreien.

Wenn das tomosynthetische 3D-Röntgenbild ausschließlich durch eine für die von der binären 3D-Primärmaske bzw. der binären 3D-Sekundärmaske nicht ausgeblendeten Voxel durchgeführte Rückprojektion (sogenannte voxelgeführte oder voxelbasierte Rückprojektion) ermittelt wird, ist dessen Berechnung signifikant beschleunigt.

Insbesondere haben erster und zweiter Projektionswinkel bezogen auf die senkrecht auf der Empfangsfläche des Röntgendetektors stehende Mittenachse entgegengesetztes Vorzeichen und sind betragsmäßig gleich und entsprechen vorzugsweise den maximal benutzten Projektionswinkeln.

Hinsichtlich der Einrichtung wird die Aufgabe gemäß der Erfindung gelöst mit einer Einrichtung mit den Merkmalen des Patentanspruches 5.

Zur weiteren Erläuterung der Erfindung wird auf das Ausführungsbeispiel der Zeichnung verwiesen. Es zeigen:

Fig. 1    eine Einrichtung gemäß der Erfindung in einer schema- tischen Prinzipdarstellung,

Fig. 2    ein tomosynthetisches Schichtbild mit im Stand der Technik bei tomosynthetischen 3D-Röntgenbildern auf-tretenden Fächer-Artefakten,

Fig. 3    ein Flussdiagramm mit dem erfindungsgemäßen Algorith- mus zum Berechnen einer 3D-Maske für das tomosynthe- tische 3D-Röntgenbild,

Fig. 4    ein Diagramm, in dem die Häufigkeit der Voxelwerte eines zur Berechnung der binären 3D-Maske aus zwei unterschiedenen Projektionswinkeln aufgenommenen 2D- Röntgenbildern erzeugten 3D-Datensatzes gegen den Vo- xelwert aufgetragen ist.

Gemäß Fig. 1 umfasst die Einrichtung, im Ausführungsbeispiel ein Mammographiegerät, eine Röntgenquelle 2, in der Regel eine Röntgenröhre, zum Erzeugen von Röntgenstrahlen 3, die ein Untersuchungsobjekt 4 durchqueren. Bei dem Untersuchungsobjekt 4 handelt es sich um eine weibliche Brust, die zwischen einer Kompressionsplatte 6 und einer Lagerplatte 8 eingebettet ist. Die das Untersuchungsobjekt 4, die Kompressionsplatte 6 und die Lagerplatte 8 durchquerenden Röntgenstrahlen 3 werden von einem großflächigen digitalen Röntgendetektor 10 empfangen, der aus einer Vielzahl von in einem matrixförmigen Array angeordneten Einzeldetektoren aufgebaut ist, und dessen Empfangsfläche 12 parallel zur Kompressionsplatte 6 und zur Lagerplatte 8 angeordnet ist.

Die Röntgenquelle 2 ist zum Untersuchungsobjekt ortsveränderbar angeordnet, und kann beispielsweise in einem begrenzten Winkelbereich $\varphi_1$, $\varphi_2$, in der Praxis etwa +25°, -25°, um eine zur Zeichenebene senkrechte Achse M in unterschiedliche Winkelpositionen j=-n ... +n geschwenkt werden, so dass vom Untersuchungsobjekt 4 2D-Röntgenbilder mit verschiedenen Projektionswinkeln $\alpha_j$ relativ zu einer senkrecht auf der Empfangsfläche 12 stehenden Mittenachse 13 erzeugt werden können. Der Winkelbereich $\varphi_1$, $\varphi_2$ muss dabei nicht symmetrisch zu dieser Mittenachse 13 angeordnet sein. Diese 2D-Röntgenbilder Bj (Einzelbilder) bzw. die diesen jeweils zugeordneten Projektions- oder Bilddaten Bj werden in einer einen Bildrechner enthaltenden Steuer- und Auswerteeinrichtung 14 durch Rekonstruktion zu einem aus einer Mehrzahl von Schichtbildern bestehenden tomosynthetischen 3D-Röntgenbild T zusammengesetzt, die auf einem Monitor 18 dargestellt werden.

Auch eine Bewegung der Röntgenquelle 2 auf einer begrenzten linearen Bahn statt des Schwenks ist zulässig, so dass die Höhendifferenz zwischen Röntgendetektor 10 und Röntgenröhre konstant bleibt. Diese lineare Bahn muss ebenfalls nicht notwendigerweise symmetrisch zur Mittenachse 13 verlaufen. Bei dieser linearen Bewegung erfolgt ein Ausrichten der Röntgenquelle 2 auf das Untersuchungsobjekt 4, so dass auch in diesem Fall vom Untersuchungsobjekt 4 in einem begrenzten Winkelbereich Einzelbilder unter verschiedenen Projektionswinkeln $\alpha_j$ aufgenommen werden.

Im Ausführungsbeispiel ist der Röntgendetektor 10 während der Schwenkbewegung der Röntgenquelle 2 ortsfest. Grundsätzlich ist es jedoch auch möglich, den Röntgendetektor 10 gemeinsam mit der Röntgenquelle 2 zu schwenken oder der Schwenkbewegung der Röntgenquelle 2 folgend linear zu verschieben.

Das nachfolgend beschriebene Verfahren ist jedoch insbesondere für Röntgeneinrichtungen mit ortsfestem Röntgendetektor 10 und eingeschränktem Winkelbereich der Projektionen von Vorteil, da bei einer solchen Anordnung das Auftreten von Fächer-Artefakten besonders ausgeprägt ist.

Die Steuerung der Winkelposition j oder im Falle einer linearen Verschiebung der Linearposition und der Ausrichtung der Röntgenquelle 2 sowie ihrer Betriebsparameter erfolgt durch Steuersignale S, die von der Steuer- und Auswerteeinrichtung 14 generiert werden.

In der Fig. 1 ist nun eine Situation eingezeichnet, bei der ein erstes 2D-Röntgenbild B1 beim ersten Projektionswinkel $\varphi_1$, beispielsweise +25° (in Fig. 1 übertrieben eingezeichnet) - im Beispiel der Figur B1 $\equiv$ B$_n$ - und ein zweites 2D-Röntgenbild B2, bei dem Projektionswinkel $\varphi_2$, beispielsweise -25° - im Beispiel der Figur B2 $\equiv$ B$_{-n}$ -, erzeugt wird. Im dargestellten Ausführungsbeispiel sind erster und zweiter Projektionswinkel $\varphi_1$, $\varphi_2$ betragsmäßig gleich, haben jedoch entgegengesetztes Vorzeichen und entsprechen zugleich den beiden Extrempositionen, die die Röntgenquelle 2 einnehmen kann. In Fig. 1 sind nun jeweils die das Untersuchungsobjekt 4 tangierenden Randstrahlen des von der Röntgenröhre jeweils emittierten Teilbündels eingezeichnet, das das Untersuchungsobjekt 4 durchquert. In der Praxis erfasst das tatsächlich von der Röntgenquelle 2 emittierte Röntgenstrahlbündel einen größeren Detektorbereich, so dass auf den Röntgendetektor 10 auch Röntgenstrahlung auftrifft - sogenannte Direktstrahlung -, die das Untersuchungsobjekt 4 nicht durchquert hat. In der Fig. ist dies durch Röntgenstrahlen veranschaulicht, die in ein durch den Winkelbereich δ bezeichnetes Raumgebiet emittiert werden.

Diejenigen Röntgenstrahlen 3, die ausgehend von der Röntgenquelle 2 in der Winkelposition j=n (Projektionswinkel ($\varphi_1$) das Untersuchungsobjekt 4 durchqueren, treffen auf den Röntgendetektor 10 in einem ersten Detektorbereich 21 auf. Die außerhalb dieses Detektorbereichs 21 liegenden Bereiche auf dem Röntgenstrahldetektor 10 sind diagnostisch irrelevante Direktstrahlungsbereiche. Dementsprechend wird das Untersuchungsobjekt 4 auf einen Detektorbereich 22 abgebildet, wenn sich die Röntgenquelle 2 in der Winkelposition j=-n befindet. Beide Detektorbereiche 21 und 22 überlagern sich in einem durch Kreuzschraffur gekennzeichneten Detektorbereich 23. Bei den Detektorbereichen, die durch die Differenz des Detektorbereiches 21 und des Detektorbereiches 23 bzw. des Detektorbereiches 22 und des Detektorbereiches 23 gebildet und in Fig. 1 durch Schrägschraffur gekennzeichnet sind, handelt es sich um Randbereiche, die in der Winkelposition j=n einem Objektbereich und in der Winkelposition j=-n einem Direktstrahlungsbereich (Winkelbereich δ) bzw. in der Winkelposition j=-n einem Objektbereich und in der Winkelposition j=n einem Direktstrahlungsbereich (Winkelbereich δ) entsprechen. Diese winkelabhängige Überlagerung von Objektbereich und Direktstrahlungsbereich erzeugt nun bei der Rekonstruktion eines tomosynthetischen 3D-Bildes die eingangs erwähnten Fächer-Artefakte.

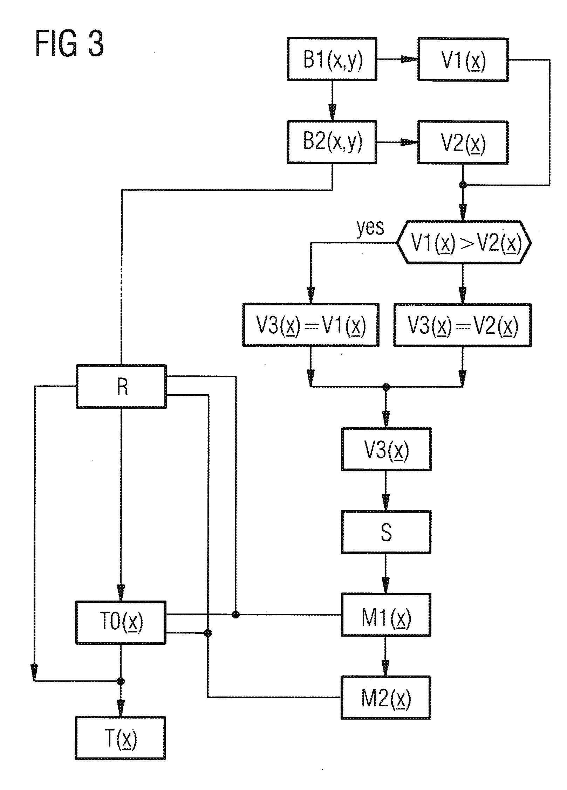

Der zum Eliminieren dieser Fächer-Artefakte gemäß der Erfindung vorgeschlagene Algorithmus ist anhand des Flussdiagrammes gemäß Fig. 3 veranschaulicht. Aus den Rohbilddaten (Grauwerten) - das sind die vom Röntgenempfänger 10 und der nachgeschalteten Elektronik bereitgestellten gegebenenfalls allenfalls durch ein binning benachbarter Pixel zusammengefassten und gemittelten digitalen Messwerte - eines ersten 2D-Röntgenbildes B1(x,y), wobei x,y die Bildkoordinaten des Detektors sind, das bei dem ersten Projektionswinkel ($\varphi_1$ aufgenommen worden ist, werden nun durch Rückprojektion erste 3D-Daten erzeugt, bei dem jedem Voxel $\underline{X}=(X_1, X_2, X_3)$ ein im Folgenden als erster Voxelwert V1($\underline{x}$) bezeichneter Intensitäts- oder Grauwertwert zugeordnet ist. Aus den Rohbilddaten des bei in einer gegenüberliegenden Position bei einem zweiten Projektionswinkel $\varphi_2$ aufgenommenen 2D-Röntgenbildes B2 (x, y) werden durch Rückprojektion zweite 3D-Daten erzeugt, indem für jedes Voxel $\underline{x}$ ein zweiter Voxelwert V2($\underline{x}$) berechnet wird.

Aus den ersten und zweiten Voxelwerten V1($\underline{x}$) bzw. V2($\underline{x}$) werden nun in einem nächsten Schritt dritte 3D-Daten bzw. Voxelwerte V3($\underline{x}$) erzeugt, indem jedem Voxel $\underline{x}$ der erste Voxelwert V1($\underline{x}$) zugeordnet wird, wenn dieser für dieses Voxel $\underline{x}$ größer als der zweite Voxelwert V2($\underline{x}$) ist. Der zweite Voxelwert V2($\underline{x}$) wird den Voxeln $\underline{x}$ zugeordnet, für die dieser größer oder gleich dem ersten Voxelwert V1($\underline{x}$) ist.

$$V3(\underline{x}) = V1(x), \text{ wenn } V1(\underline{x}) > V2(\underline{x})$$

$$V3(\underline{x}) = V2(x), \text{ wenn } V1(\underline{x}) \leq V2(\underline{x})$$

Auf diese Weise werden die ersten Voxelwerte V1($\underline{x}$) beibehalten oder ersetzt und es entsteht ein durch dritte Voxelwerte V3($\underline{x}$) gebildeter 3D-(Volumen)Datensatz, bei dem die auf den kreuzschraffierten Detektorbereich 23 projizierten Voxel $\underline{x}$ Voxelwerte V3($\underline{x}$) aufweisen, die sich signifikant von den durch Direktstrahlung gebildeten hohen Voxelwerten V3($\underline{x}$) der Umgebung unterscheiden. Mit anderen Worten: Nur diejenigen Voxel $\underline{x}$ werden nicht einem Direktstrahlungsbereich sondern dem Objektbereich zugeordnet, die weder beim ersten Projektionswinkel ($\varphi_1$ noch beim zweiten Projektionswinkel $\varphi_2$ aus Direktstrahlungsbereichen rekonstruiert werden. Der auf diese Weise identifizierte Objektbereich ist dann zwangsläufig der Detektorbereich, der für den Fall, dass die Winkelpositionen $\varphi_1$, $\varphi_2$ den beiden symmetrisch zur senkrecht auf Empfangsfläche des Detektors stehenden Mittenachse befindlichen Extrempositionen der Röntgenquelle entsprechen, ausschließlich Bilddaten enthält, die durch Röntgenstrahlung erzeugt sind, die das Untersuchungsobjekt durchquert haben. Grundsätzlich ist es aber auch möglich, Winkelpositionen $\varphi_1$, $\varphi_2$ zu wählen, die nicht den gegenüberliegenden Extrempositionen entsprechen, falls die dann mit zunehmenden Abstand von den Extrempositionen zunehmend entstehenden Fächer-Artefakte in Kauf genommen werden sollen, um möglichst wenig Information über den äußersten Randbereich des Untersuchungsobjektes zu verlieren.

Obwohl es grundsätzlich möglich ist, im Rahmen des vorstehend erläuterten Algorithmus drei vollständige, aus den ersten, zweiten und dritten Voxelwerten V1-3($\underline{x}$) aufgebaute 3D-Datensätze zu erzeugen und zu speichern, ist es aus Speicherplatzgründen zweckmäßig, die ersten Voxelwerte V1($\underline{x}$) punktweise durch die dritten Voxelwerte V3($\underline{x}$) zu ersetzen, so dass praktisch nur Speicherplatz für einen einzigen 3D-Datensatz benötigt wird.

In einem darauffolgenden Schritt wird im Histogramm des aus den dritten Voxelwerten V3($\underline{x}$) gebildeten 3D-Datensatzes mit bekannten Methoden der Bildverarbeitung ein Schwellwert identifiziert, mit dem der Objektbereich vom Direktstrahlungsbereich getrennt wird. Alle Voxel $\underline{x}$ deren dritte Voxelwerte V3($\underline{x}$) kleiner sind als dieser Schwellwert S, werden dem Objektbereich zugeordnet und es wird eine erste binäre 3D-Maske M1($\underline{x}$) für das tomosynthetische 3D-Röntgenbild gebildet, die für alle Voxel $\underline{x}$ den Wert 0 erhält, deren dritter Voxelwert V3($\underline{x}$) den Schwellwert S überschreitet. Alle übrigen Voxel $\underline{x}$ haben in der ersten binären 3D-Maske M1($\underline{x}$) den Wert 1. Diese erste 3D-Maske M1($\underline{x}$) kann nun auf das mit bekannten Rekonstruktionsverfahren R aus einer Vielzahl von 2D-Röntgenbildern B1, B2,... berechnete tomosynthetische 3D-Roh-Röntgenbild T0($\underline{x}$), das noch mit Fächer-Artefakten behaftet ist, angewendet werden, um auf diese Weise ein artefaktfreies oder -reduziertes tomosynthetisches 3D-Röntgenbild T(x) zu erzeugen, aus dem Fächer-Artefakte weitgehend eliminiert sind.

Aus der binären 3D-Primärmaske M1($\underline{x}$) kann außerdem durch Methoden der Bildverarbeitung eine gefilterte oder geglättete zweite binäre 3D-Maske M2($\underline{x}$) erzeugt werden, die anstelle der ersten 3D-Maske M1($\underline{x}$) auf das tomosynthetische 3D-Roh-Röntgenbild T0(x) angewendet werden kann. Das erfindungsgemäße Verfahren ist somit auch auf bereits vorher erstellte tomosynthetische 3D-Röntgenbilder anwendbar, wenn die diesen zugrundeliegenden Rohbilddaten der 2D-Röntgenbilder B(x, y) zumindest für zwei einander gegenüberliegende Winkelpositionen der Röntgenquelle bekannt sind.

Bei der sogenannten voxelgeführten (voxel driven) Rückprojektion ist es auch möglich, eine der 3D-Masken M1, M2 bereits bei der Rückprojektion einzusetzen, d. h. nicht erst auf ein fertiggestelltes tomosynthetisches 3D-Röntgenbild anzuwenden. In diesem Fall braucht die Rekonstruktion R nur für eine durch die 3D-Maske M1 oder M2 begrenzte Anzahl von Voxeln durchgeführt zu werden und es entsteht ohne Umwege das von Fächer-Artefakten befreite tomosynthetische 3D-Röntgenbild T($\underline{x}$).

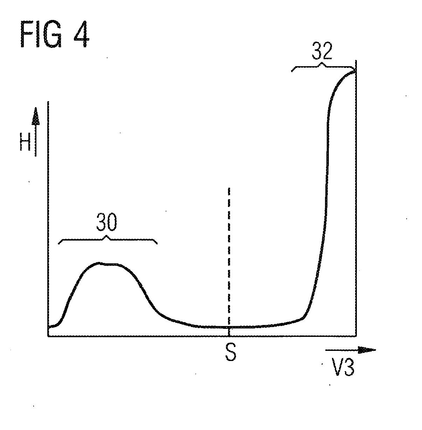

In Fig. 4 ist exemplarisch ein Histogramm eines dritten 3D-Datensatzes dargestellt, in dem die Häufigkeit H gegen den dritten Voxelwert V3 aufgetragen ist. Diesem Histogramm ist zu entnehmen, dass es zwei deutlich voneinander getrennte Voxelwertbereiche 30,32 gibt, anhand der er sich eindeutig identifizieren lässt, ob ein Voxelwert V3 einem Voxel außerhalb (Voxelwertbereich 32) des Untersuchungsobjektes oder innerhalb (Voxelwertbereich 30) des Untersuchungsobjektes zugeordnet ist. Anhand dieses Histogrammes lässt sich mit Methöden der digitalen Bildverarbeitung automatisch der Schwellwert S berechnen, der für die Erzeugung der ersten binären 3D-Primärmaske M1($\underline{x}$) herangezogen wird.

**Patentansprüche**

1. Verfahren zum Erzeugen eines tomosynthetischen 3D-Röntgenbildes (T), bei dem von einem Untersuchungsobjekt (4) mit verschiedenen Projektionswinkeln ($\alpha_j$) in einem begrenzten Winkelbereich ($\varphi_1+\varphi_2$) mit einer Röntgenquelle (2) und einem digitalen Röntgendetektor (10) eine Mehrzahl von digitalen 2D-Röntgenbildern (Bj) aufgenommen wird, mit folgenden Verfahrensschritten:

   a) aus den Bilddaten eines mit einem ersten Projektionswinkel ($\varphi_1$) aufgenommenen ersten 2D-Röntgenbildes (B1($\underline{x}$)) und den Bilddaten eines mit einem zweiten Projektionswinkel ($\varphi_2$) aufgenommenen zweiten 2D-Röntgenbildes (B2($\underline{x}$)) wird durch Rückprojektion jedem Voxel (x) ein erster bzw. zweiter Voxelwert (V1($\underline{x}$), V2($\underline{x}$)) zugeordnet,
   b) aus den ersten und zweiten Voxelwerten (V1($\underline{x}$), V2($\underline{x}$)) wird ein 3D-Datensatz erzeugt, bei dem jedem Voxel (x) ein dritter Voxelwert (V3($\underline{x}$)) zugeordnet ist, der **dadurch** festgelegt ist, dass jedem Voxel ($\underline{x}$) als dritter Voxelwert (V3($\underline{x}$)) der erste Voxelwert (V1($\underline{x}$)) zugeordnet wird, wenn dieser größer als der zweite Voxelwert (V2($\underline{x}$)) ist, und jedem Voxel (x) als dritter Voxelwert (V3($\underline{x}$)) der zweite Voxelwert (V2($\underline{x}$)) zugeordnet wird, wenn dieser größer als der erste Voxelwert (V1($\underline{x}$)) ist,
   c) aus der Häufigkeitsverteilung der dritten Voxelwerte (V3($\underline{x}$)) wird ein Schwellwert (S) ermittelt, mit dem eine erste binäre 3D-Maske (M1($\underline{x}$)) für das tomosynthetische 3D-Röntgenbild gebildet wird, die für alle Voxel ($\underline{x}$) den Wert 0 erhält, deren dritter Voxelwert (V3($\underline{x}$)) den Schwellwert (S) überschreitet,
   d) das tomosynthetische 3D-Röntgenbild wird unter Zuhilfenahme der ersten binären 3D-Maske (M1($\underline{x}$)) oder einer aus dieser durch Methoden der Bildverarbeitung abgeleiteten, gefilterten oder geglätteten zweiten binären 3D-Maske (M2($\underline{x}$)) erzeugt.

2. Verfahren nach Anspruch 1, bei dem mit der binären 3D-Maske (M1($\underline{x}$), M2($\underline{x}$)) ein fertiges artefaktbehaftetes rekonstruiertes tomosynthetisches 3D-Röntgenbild bearbeitet wird.

3. Verfahren nach Anspruch 1, bei dem das tomosynthetische 3D-Röntgenbild ausschließlich durch eine für die von der ersten bzw. zweiten binären 3D-Maske (M1($\underline{x}$), M2($\underline{x}$)) nicht ausgeblendeten Voxel (x) durchgeführte Rückprojektion ermittelt wird.

4. Verfahren nach einem der vorhergehenden Ansprüche, bei dem erster und zweiter Projektionswinkel ($\varphi_1$, $\varphi_2$) bezogen auf die senkrecht auf der Empfangsfläche (12) des Röntgendetektors (10) stehenden Mittenachse (13) entgegengesetztes Vorzeichen haben und betragsmäßig gleich sind.

5. Einrichtung zum Erzeugen eines tomosynthetischen 3D-Röntgenbildes (T) mit einer in einem begrenzten Detektorbereich zu einem Untersuchungsobjekt (4) ortsveränderbar angeordneten Röntgenquelle (2) und mit einem digitalen Röntgendetektor (10) zum Aufnehmen von digitalen 2D-Röntgenbildern (Bj) mit verschiedenen Projektionswinkeln ($\alpha_j$) sowie mit einer Auswerteeinrichtung (14) zum Verarbeiten der vom Röntgendetektor (10) bereitgestellten Detektorsignale, **gekennzeichnet durch** eine darin implementierte Software zum Durchführen eines Verfahrens nach einem der vorhergehenden Ansprüche.

**Claims**

1. Method for creating a tomosynthetic 3D X-ray image (T), in which a plurality of digital 2D X-ray images (Bj) of an object under examination (4) is recorded with different angles of projection ($\alpha_j$) in a restricted angular range ($\varphi_1+\varphi_2$) with an X-ray source (2) and a digital X-ray detector (10), with the following method steps:

   a) from the image data of a first 2D X-ray image (B1($\underline{x}$)) recorded with a first angle of projection ($\varphi_1$) and the image data of a second 2D X-ray image (B2($\underline{x}$)) recorded with a second angle of projection ($\varphi_2$), each voxel ($\underline{x}$) is assigned a first or a second voxel value (V1($\underline{x}$), V2($\underline{x}$)) by back projection,

b) a 3D dataset is created from the first and the second voxel values (V1($\underline{x}$), V2($\underline{x}$)), in which each voxel ($x$) is assigned a third voxel value (V3($\underline{x}$)), which is defined by each voxel ($\underline{x}$) being assigned the first voxel value (V1($\underline{x}$)) as the third voxel value (V3($\underline{x}$)) if said value is greater than the second voxel value (V2($\underline{x}$)), and each voxel ($\underline{x}$) being assigned the second voxel value (V2($\underline{x}$)) as the third voxel value (V3($\underline{x}$)) if said value is greater than the first voxel value (V1($\underline{x}$)),

c) a threshold value (S) is determined from the frequency distribution of the third voxel values (V3($\underline{x}$)), with which a first binary 3D mask (M1($\underline{x}$)) is formed for the tomosynthetic 3D X-ray image, which is given the value 0 for all voxels ($\underline{x}$), of which the third voxel value (V3($\underline{x}$)) exceeds the threshold value (S),

d) the tomosynthetic 3D X-ray image is created with the aid of the first binary 3D mask (M1($\underline{x}$)) or of a second filtered or smoothed binary 3D mask (M2($\underline{x}$)) derived therefrom by image processing methods.

2. Method according to claim 1, in which with the binary 3D mask (M1($\underline{x}$), M2($\underline{x}$)) a completed, artifact-affected reconstructed tomosynthetic 3D X-ray image is processed.

3. Method according to claim 1, in which the tomosynthetic 3D X-ray image is determined exclusively by a back projection carried out for the voxels ($\underline{x}$) not masked out by the first or second 3D mask (M1($\underline{x}$), M2($\underline{x}$)).

4. Method according to one of the preceding claims, in which the first and second projection angle ($\varphi_1 + \varphi_2$) have opposing leading signs and are equivalent in amount in relation to the central axis (13) of the receive surface (12).

5. Device for creating a tomosynthetic 3D X-ray image (T) with an X-ray source (2) of which the location is able to be changed in a restricted detector range in relation to an object under examination (4) and with a digital X-ray detector (10) to record digital 2D X-ray images (Bj) at different angles of projection ($\alpha_j$) and also with an evaluation device (14) for processing the detector signals provided by the X-ray detector (10), **characterised by** software implemented therein for carrying out a method according to one of the previous claims.

**Revendications**

1. Procédé pour générer une image radiotomographique de synthèse en 3D (T), avec lequel une pluralité d'images radio numériques en 2D (B$_j$) sont faites d'un objet d'étude (4) avec différents angles de projection ($\alpha_j$) dans une plage d'angle ($\varphi_1 + \varphi_2$) limitée avec une source de rayons X (2) et un détecteur de rayons X (10) numérique, présentant les étapes suivantes :

a) à partir des données d'image d'une première image radio en 2D (B1($\underline{x}$)) enregistrée avec un premier angle de projection ($\varphi_1$) et des données d'image d'une seconde image radio en 2D (B2($\underline{x}$)) enregistrée avec un second angle de projection ($\varphi2$), une première ou une seconde valeur voxel (V1($\underline{x}$), V2($\underline{x}$)) est attribuée par rétroprojection à chaque voxel ($\underline{x}$),

b) à partir des premières et secondes valeurs voxel (V1($\underline{x}$), V2($\underline{x}$)), on génère un ensemble de données en 3D, dans lequel une troisième valeur voxel (V3($\underline{x}$)) est attribuée à chaque voxel ($\underline{x}$), laquelle valeur est définie par le fait que la première valeur voxel (V1($\underline{x}$)) est attribuée à chaque voxel ($\underline{x}$) en tant que troisième valeur voxel (V3($\underline{x}$)) lorsque cette valeur est supérieure à la seconde valeur voxel (V2($\underline{x}$)), et la seconde valeur voxel (V2($\underline{x}$)) est attribuée à chaque voxel ($\underline{x}$) comme troisième valeur voxel (V3($\underline{x}$)) lorsque cette valeur est supérieure à la première valeur voxel (V1($\underline{x}$)),

c) à partir de la répartition de fréquence des troisièmes valeurs voxel (V3($\underline{x}$)), on détermine une valeur seuil (S), avec laquelle un premier masque en 3D binaire (M1($\underline{x}$)) est formé pour l'image radiotomographique de synthèse en 3D, qui a la valeur 0 pour tous les voxels ($\underline{x}$) dont la troisième valeur voxel (V3($\underline{x}$)) dépasse la valeur seuil (S),

d) l'image radiotomographique de synthèse en 3D est générée à l'aide du premier masque en 3D binaire (M1($\underline{x}$)) ou d'un second masque en 3D binaire (M2($\underline{x}$)) déduit du premier masque par des méthodes de traitement d'image, filtrée ou lissé.

2. Procédé selon la revendication 1, dans lequel une image radiotomographique de synthèse en 3D achevée, entachée d'artéfact et reconstituée est traitée avec le masque en 3D binaire (M1($\underline{x}$), M2($\underline{x}$)).

3. Procédé selon la revendication 1, avec lequel l'image radiotomographique de synthèse en 3D est déterminée uniquement par une rétroprojection qui est effectuée pour les voxels ($\underline{x}$) qui ne sont pas supprimés par le premier ou le second masque en 3D binaire (M1($\underline{x}$), M2($\underline{x}$)).

4. Procédé selon l'une quelconque des revendications précédentes, avec lequel le premier et le second angle de projection ($\varphi_1$, $\varphi_2$) ont un signe opposé par rapport à l'axe central (13) disposé perpendiculairement à la surface de réception (12) du détecteur de rayons X (10) et sont identiques au niveau de la valeur.

5. Equipement pour générer une image radiotomographique de synthèse en 3D (T) comprenant une source de rayons X (2) disposée de façon variable en position dans une zone de détecteur limitée par rapport à un objet d'étude (4) et un détecteur de rayons X (10) numérique pour l'enregistrement d'images radio numériques en 2D ($B_j$) avec des angles de projection ($\varphi$) différents et un dispositif d'analyse (14) pour le traitement des signaux de détecteur mis à disposition par le détecteur de rayons X (10), **caractérisé par** un logiciel mis en place à l'intérieur pour la mise en oeuvre d'un procédé selon l'une quelconque des revendications précédentes.

8

FIG 1

$\varphi 2$

$\alpha_j$

$\varphi_1$

2

$j = -n$

$j = n$

13

$\delta$

M

3

$\delta$

3

4

6

S

8

12

23

10

21

14

18

22

Bj, T

FIG 2

TS

4

A

# FIG 3

FIG 4

**IN DER BESCHREIBUNG AUFGEFÜHRTE DOKUMENTE**

**In der Beschreibung aufgeführte Patentdokumente**

- US 20050135559 A1

**In der Beschreibung aufgeführte Nicht-Patentliteratur**

- **Godfrey DJ.** Digital x-ray tomosynthesis: current state of the art and clinical potential. *Physics in Medicine and Biology,* 2003, vol. 48, R65-R106